# EUROPÄISCHE PATENTSCHRIFT

(11) **EP 0 688 187 B1**
(45) Veröffentlichungstag und Bekanntmachung des Hinweises auf die Patenterteilung: **03.06.1998**
(21) Anmeldenummer: 94911050.6
(22) Anmeldetag: 10.03.1994
(51) Int. Cl.: A61B 17/28

(54) **MEDIZINISCHE ZANGE**
MEDICAL PLIERS
PINCE MEDICALE

(30) Priorität: 10.03.1993 DE 4307539
(43) Veröffentlichungstag der Anmeldung: 27.12.1995
(73) Patentinhaber: Karl Storz GmbH & Co., 78532 Tuttlingen (DE)
(72) Erfinder: STORZ, Karl, D-78532 Tuttlingen (DE)
(74) Vertreter: Münich, Wilhelm, Dr.
(86) Internationale Anmeldenummer: DE9400247
(87) Internationale Veröffentlichungsnummer: WO9420034

(56) Entgegenhaltungen:
- EP-A- 0 484 671
- WO-A-92/11812
- DE-A- 3 741 879
- DE-U- 8 905 099
- DE-U- 9 007 356
- DE-U- 9 202 132
- DE-U- 9 214 059
- US-A- 2 113 246
- US-A- 4 674 501

## Beschreibung

### Technisches Gebiet

Die Erfindung bezieht sich auf eine medizinische Zange insbesondere für die Verwendung bei endoskopischen Eingriffen gemäß dem Oberbegriff des Patentanspruchs 1.

Aus dem Dokument DE-U-90 07 356 ist eine gattungsgemäße medizinische Zange insbesondere für die Verwendung bei endoskopischen Eingriffen bekannt. Diese Zange weist ein Handstück, das einen feststehenden und einen beweglichen Handgriff aufweist, ein Außenrohr, das mit dem feststehenden Handgriff verbunden ist, und ein in dem Außenrohr verschiebbar geführtes Zugelement auf, das wenigstens eines der beiden Zangenelemente des Zangenmauls betätigt, und das mit dem beweglichen Handgriff verbunden ist.

### Stand der Technik

Bekannte gattungsgemäße Zangen weisen ein Handstück mit einem feststehenden und einem beweglichen Handgriff auf. Mit dem feststehenden Handgriff ist ein Außenrohr verbunden, in dem eine Zugstange verschiebbar geführt ist, die wenigstens eines der beiden Zangenelemente betätigt, und die mit dem beweglichen Handgriff verbunden ist.

Aus einer Reihe von Gründen - genannt seien an dieser Stelle nur die Reinigung und Sterilisierung sowie der Austausch defekter Teile bzw. die Umrüstung insbesondere der Zangenelemente - wäre es wünschenswert, gattungsgemäße medizinische Zangen leicht zerlegen zu können. Die bekannten Zangen sind jedoch nicht oder nur mit Schwierigkeiten zu zerlegen. Darüberhinaus bereitet der Zusammenbau der zerlegten Zangen Schwierigkeiten.

Aus dem Dokument DE-U-89 05 099 ist es zwar bereits bekannt, eine medizinische Zange - die allerdings anderer Gattung als im Oberbegriff des Patentanspruchs 1 verausgesetzt ist - mit einer Bajonetteverbindung auszustatten, um sie für den Reinigungsvorgang zerlegen und anschließend wieder leicht zusammensetzen zu können.

### Darstellung der Erfindung

Der Erfindung liegt die Aufgabe zugrunde, eine medizinische Zange insbesondere für die Verwendung bei endoskopischen Eingriffen gemäß dem Oberbegriff des Patentanspruchs 1 derart weiterzubilden, daß die Zange leicht zu zerlegen ist, wobei nach ihrem Zusammenbau ohne weitere Maßnahmen die korrekte Funktion der Zange sichergestellt sein muß.

Eine erfindungsgemäße Lösung dieser Aufgabe ist im Patentanspruch 1 angegeben. Weiterbildungen der Erfindung sind Gegenstand der Ansprüche 2 folgende.

Erfindungsgemäß ist auf dem distalen Ende der Zugstange relativ zur Zugstange verschiebbar ein Bajonettelement gelagert, das in einen Bajonetteinsatz in dem Außenrohr einsetzbar ist, und an dem wenigstens eines der Zangenelemente angelenkt ist. Durch die Bajonettverbindung zwischen Außenrohr und Zugstange ist nicht nur eine leichte Entnahme der Zugstange aus dem Außenrohr sichergestellt, sondern es ist auch durch die durch den Bajonetteingriff vorgegebene Drehlage-Ausrichtung gewährleistet, daß beim Zusammenenbau ein korrektes Einsetzen der Zugstange in das Außenrohr erfolgt, und die beiden Elemente sicher miteinander verbunden sind. Durch das im Außenrohr vorgesehene federbelastete Klemmelement, das bei aufgesetztem Handstück formschlüssig in die Zugstange eingreift, wird sichergestellt, daß die Bajonetteverbindung im zusammengebauten Zustand der Zange nicht unabsichtlich gelöst werden kann.

Bei einer Weiterbildung der Erfindung ist das Außenrohr mit eingesetzter Zugstange mit dem Handstück über ein Verbindungselement verbindbar, wobei das proximale Ende der Zugstange in eine federbelastete Raste in dem beweglichen Handgriff des Handstücks einrastet. Diese Ausbildung stellt die einwandfreie Verbindung mit dem Handstück sicher, wobei sämtliche Freiheitsgrade, beispielsweise für die Drehung des Außenrohrs mit eingesetzter Zugstange, erhalten bleiben.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1: eine medizinische Zange teilweise geschnitten,
- Fig. 2: das Detail x aus Fig. 1,
- Fig. 3: die distale Verbindung Zugstange/Außenrohr, und
- Fig. 4: die proximale Verbindung Zugstange/Außenrohr.

### Beschreibung eines Ausführungsbeispiels

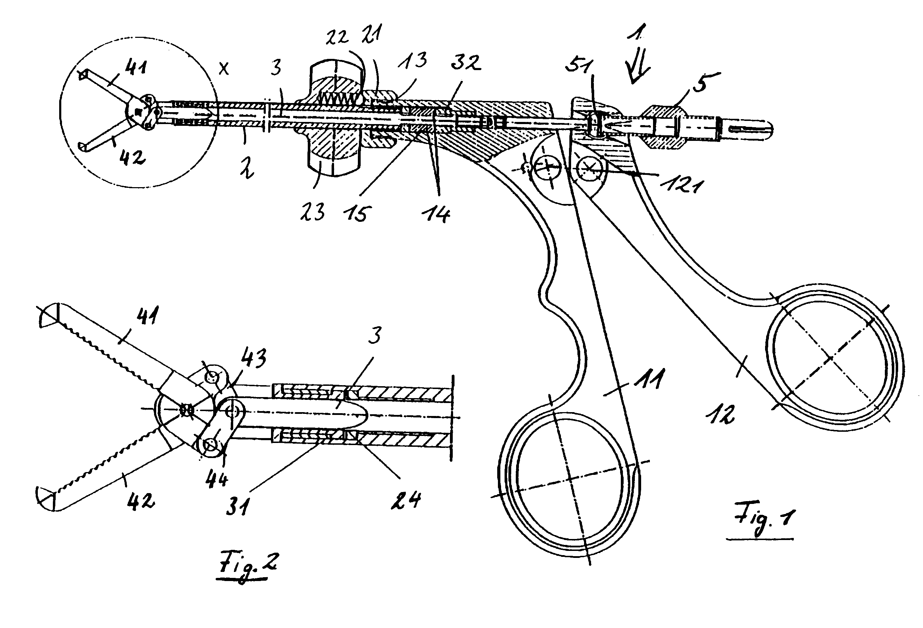

Fig. 1 zeigt in einer Seitenansicht bzw. in einem teilweisen Schnitt eine erfindungsgemäß ausgebildete medizinische Zange insbesondere für die Verwendung bei endoskopischen Eingriffen. Die Zange weist ein Handstück 1 mit einem feststehenden Handgriff 11 und einem beweglichen Handgriff 12 auf. Der bewegliche Handgriff 12 ist an dem feststehenden Handgriff 11 über ein nicht näher dargestelltes Gelenk 121 schwenkbar angelenkt.

Der feststehende Handgriff 11 weist an seinem dem distalen Ende der Zange zugekehrten Ende ein Schraubgewinde 13 (Außengewinde) auf, auf das ein Schraubelement 21 mit einem Innengewinde 21' aufgeschraubt ist. Das Schraubelement 21 ist drehbar mit einem Griffteil 23 verbunden, das wiederum fest mit einem Außenrohr 2 der Zange 1 verbunden ist.

Um definierte Verdrehungen bzw. Drehstellungen des Griffteils 23 bzw. des Außenrohrs 2 relativ zu dem Schraubelement 21 bzw. dem feststehenden Handgriff 11 einstellen zu können, ist wenigstens eine von einer Feder 22' belastete Kugel 22 vorgesehen, die in entsprechende Ausnehmungen in dem Schraubelement 21 eingreift bzw. einrastet. Durch die Zahl und den Winkelabstand der Ausnehmungen ist die Zahl der rastbaren Drehstellungen zwischen dem Außenrohr 2 und dem Schraubelement 21 vorgegeben.

Selbstverständlich ist es möglich, anstelle einer Kugelrastung eine andere, eine definierte Winkelstellung erlaubende Rastverbindung vorzusehen. Weiterhin kann auch auf eine Rastung verzichtet werden, so daß eine Relativ-Drehstellung zwischen dem Handgriff 11 und dem Außenrohr 2 beispielsweise durch einen Reibschluß fixiert wird.

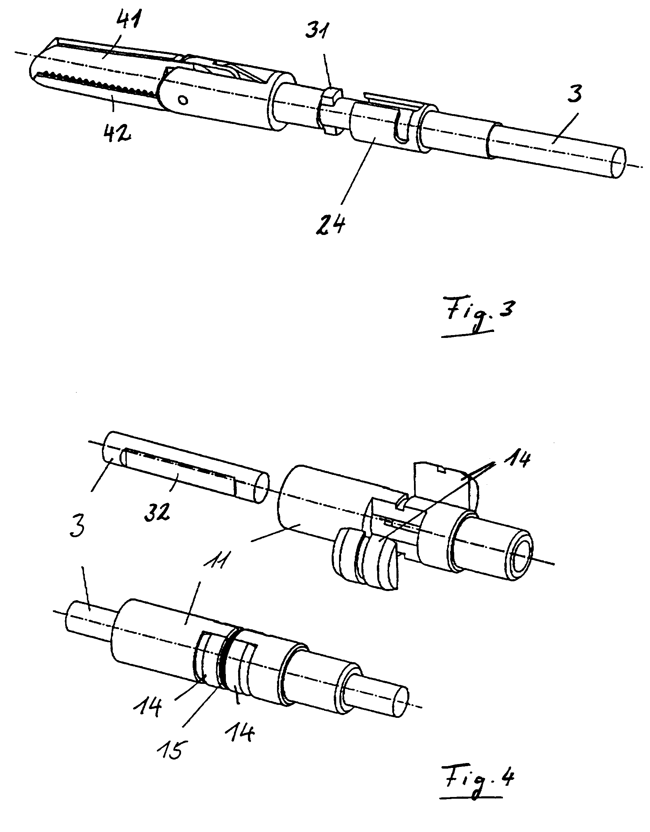

In dem Außenrohr 2 ist eine Zugstange 3 derart verschiebbar geführt, daß sie einer Drehung des Außenrohrs 2 relativ zum feststehenden Handgriff 11 folgt. Dies wird in Verbindung mit den Figuren 2 und 3 noch erläutert. Die Zugstange 3 ist mit dem beweglichen Handgriff 12 verbunden. Durch eine Verschiebung der Zugstange 3 werden am distalen Ende der Zange vorgesehene Zangenelemente 41 und 42, die das sog. Zangenmaul 4 bilden, in einer weiter unten beschriebenen Weise betätigt, d.h. gespreizt und zusammengezogen.

Weiterhin können durch die Drehbewegung die Zangenelemente 41 und 42 entsprechend dem jeweiligen Anwendungsfall relativ zum dem Handstück 1 bezüglich ihrer Drehlage eingestellt werden.

Fig. 2 zeigt zur Erläuterung der Betätigung der beweglichen Zangenelemente 41 und 42 das Detail x aus Fig. 1.

Fig. 3 zeigt in einer perspektivischen Darstellung die Bajonettverbindung zwischen Zugstange 3 und Außenrohr 2.

Auf dem distalen Ende der Zugstange 3 ist relativ zur Zugstange verschiebbar ein Bajonettelement 31 gelagert, das in einen Bajonetteinsatz 24 in dem Außenrohr 2 einsetzbar ist. An dem Bajonettelement 31 sind die beiden Zangenelemente 41 und 42 angelenkt. Die Betätigung der beiden Zangenelemente 41 und 42 erfolgt über Schwenkelemente 43 und 44 (Fig. 2). Durch den Bajonetteingriff zwischen den Elementen 31 und 24 ist das Bajonettelement 31 relativ zum Außenrohr festgelegt, so daß durch eine Verschiebung der Zugstange 3 die Zangenelemente 41 und 42 betätigt, d.h. gespreizt bzw. zusammengezogen werden können. Gleichzeitig folgt die Zugstange 3 einer Drehung des Außenrohrs 2.

Selbstverständlich sind auch alle anderen Ausbildungen des sog. Zangenmauls möglich, wie sie aus dem Stand der Technik bekannt sind. Insbesondere ist es möglich, das Zangenmaul, d.h. die Elemente 41 und 42 so auszubilden, daß ein Element fest mit dem Bajonettelement 31 verbunden ist, und somit bei einem Öffnen des Zangenmauls nicht geschwenkt wird, während das andere Element durch eine Verschiebung der Zugstange geschwenkt wird.

Um ein Torsion der vergleichsweise dünnen Zugstange 3 bei einer Relativverdrehung zwischen Außenrohr 2 und Handstück 1 zu vermeiden, ist die Zugstange 3 an ihrem proximalen Ende in der in Fig. 4 näher dargestellten Weise gelagert:

Wie Fig. 4 zeigt, weist die Zugstange 3 an ihrem proximalen Endbereich zwei sich gegenüberliegende Abflachungen 32 auf, mit denen sie in ein Klemmelement 14 in dem proximalen Endbereich des Außenrohrs 2 einsetzbar ist. Das Bezugszeichen 15 bezeichnet einen Federring, der die beiden Hälften des Klemmelements 14 in Richtung auf die Abflachungen 32 der Zugstange 3 vorspannt.

Das proximale Ende der Zugstange 3 ist weiterhin so ausgebildet, daß es in eine federbelastete Raste 51 in dem beweglichen Handgriff 12 des Handstücks 1 einrastet. Hierzu kann die Zugstange 3 an ihrem proximalen Ende kugelförmig ausgebildet sein.

Die Raste 51 ist durch ein Betätigungselement 5 derart in Richtung der Längsachse der Zugstange 3 verschiebbar, daß die Öffnung des Zangenmauls bei einer bestimmten Stellung des beweglichen Handgriffs 12 relativ zum feststehenden Handgriff 11 einstellbar ist.

Vorstehend ist die Erfindung anhand eines Ausführungsbeispiels beschrieben worden, wobei selbstverständlich die verschiedensten Abwandlungen möglich sind:

Beispielsweise ist es möglich, durch eine nicht dargestellte Feder den beweglichen Handgriff 12 in die Stellung zu beaufschlagen, in der die Zangenelemente 41 und 42 geschlossen sind.

Weiterhin können die beiden Handgriffe 11 und 12 durch eine an sich bekannte sägezahnartige Rastung relativ zueinander festgelegt werden.

Bei einer weiteren vorteilhaften Ausgestaltung besteht das Außenrohr aus einem metallischen Innenrohr und einem aus einem isolierendem Material bestehenden Hüllrohr. Diese Ausführung der Zange kann beispielsweise als HF-Resektoskop eingesetzt werden. Von besonderem Vorteil ist es, wenn das aus einem isolierendem Material bestehende Hüllrohr über eine Bajonettverbindung mit der eigentlichen Zange verbunden ist.

Das patientennahe Ende des Metallschaftes ist gegen Strom-schäden in der Harnröhre mit einem Endstück aus einem isolierendem Werkstoff gesichert. Durch die bei Anwendung der Hochfrequenzströme auftretende Funkenbildung kommt es des öfteren zur Beschädigung des Endstücks. Das Endstück ist deshalb reparaturanfällig und führt zu Ärgernissen im praktischen Betrieb.

Bei einer erfindungsgemäßen Weiterbildung des Gegenstandes der Erfindung besteht der Schaft des Resektoskops aus zwei zerlegbaren Teilen, nämlich dem inneren metallischen Teil und dem äußeren Teil aus einem nicht leitenden Werkstoff. Der äußere Teil ist durch einen Lock-Verschluß bzw. eine Bajonett-Verbindung mit der Zange Verbund.

Bevorzugt überragt das patientennahe Ende aus nicht leitendem Werkstoff den metallischen Teil um einige Millimeter, so daß keine Schädigungen durch Hochfrequenzströme auftreten. Der preislich nicht aufwendige Teil aus nicht leitendem Werkstoff kann für einmaligen Gebrauch bestimmt sein, er kann aber auch ein wiederverwendbarer Teil sein, der beispielsweise autoklaviert werden kann.

In jedem Falle erlaubt der erfindungsgemäße Aufbau das leichte Reinigen und/oder austauschen von Teilen sowie das "Wieder-Zusammensetzen" der Zange, ohne daß es zu Funktionsstörungen kommen könnte.

## Patentansprüche

1. Medizinische Zange insbesondere für die Verwendung bei endoskopischen Eingriffen, mit
- einem Handstück (1), das einen feststehenden und einen beweglichen Handgriff (11,12)aufweist,
- einem Außenrohr (2), das mit dem feststehenden Handgriff (11) verbunden ist, und
- einer in dem Außenrohr (2) verschiebbar geführten Zugstange (3), die wenigstens eines der beiden Zangenelemente (41,42) des Zangenmauls betätigt und die mit dem beweglichen Handgriff (12) verbunden ist,
dadurch **gekennzeichnet,** daß auf dem distalen Ende der Zugstange (3) relativ zur Zugstange verschiebbar ein Bajonettelement (31) gelagert ist, das in einen Bajonetteinsatz (24) in dem Außenrohr (2) einsetzbar ist, und an dem wenigstens eines der Zangenelemente (41,42) angelenkt ist, und
daß im Außenrohr (2) ein federbelastetes Klemmelement (14,15) vorgesehen ist, das bei aufgesetztem Handstück formschlüssig in die Zugstange (3) eingreift.

2. Zange nach Anspruch 1,
dadurch **gekennzeichnet,** daß das Außenrohr (2) mit eingesetzter Zugstange (3) mit dem Handstück (1) über ein Verbindungselement (13,21) verbindbar ist, wobei das proximale Ende der Zugstange (3) in eine Raste (51) in den beweglichen Handgriff (12) des Handstücks (1) einrastet.

3. Zange nach Anspruch 2,
dadurch **gekennzeichnet,** daß die Raste (51) an den beweglichen Handgriff (12) durch ein Betätigungselement (5) derart verschiebbar ist, daß die Öffnung des Zangenmauls (4) einstellbar ist.

4. Zange nach einem der Ansprüche 1 bis 3,
dadurch **gekennzeichnet,** daß die Zugstange (3) an ihrem proximalen Endbereich zwei sich gegenüberliegende Abflachungen (32) aufweist, in die das federbelastete Klemmelement (14, 15) des Außenrohrs (2) formschlüssig eingreift.

5. Zange nach einem der Ansprüche 1 bis 4,
dadurch **gekennzeichnet,** daß die Zugstange (3) an ihrem proximalen Ende kugelförmig ausgebildet ist, mit dem sie in die Aufnahme der federbelasteten Raste (51) einsetzbar ist.

6. Zange nach einem der Ansprüche 1 bis 5,
dadurch **gekennzeichnet,** daß eine Feder den beweglichen Handgriff (12) in die Stellung beaufschlagt, in der die Zangenelemente (41,42) geschlossen sind.

7. Zange nach einem der Ansprüche 1 bis 6,
dadurch **gekennzeichnet,** daß das Verbindungselement, das das Außenrohr (2) mit dem Handstück (1) verbindet, ein Schraubelement (13,21) ist.

8. Zange nach einem der Ansprüche 1 bis 7,
dadurch **gekennzeichnet,** daß das Außenrohr (2) relativ zu dem auf dem Handstück (1) angebrachten Teil (21) des Verbindungselements drehbar ist.

9. Zange nach Anspruch 8,
dadurch **gekennzeichnet,** daß definierte Verdrehstellungen zwischen dem Außenrohr (2) und dem Handstück (1) einstellbar sind.

10. Zange nach Anspruch 9,
dadurch **gekennzeichnet,** daß eine Kugel/Rastverbindung die Verdrehstellungen definiert.

11. Zange nach einem der Ansprüche 1 bis 10,
dadurch **gekennzeichnet,** daß beide Zangenelemente (41,42) beweglich sind.

12. Zange nach Anspruch 11,
dadurch **gekennzeichnet**, daß die Zangenelemente (41,42) mit der Zugstange über Schwenkelemente (43,44) verbunden sind.

13. Zange nach einem der Ansprüche 1 bis 12,
dadurch **gekennzeichnet,** daß das Außenrohr (2) aus einem metallischen Innenrohr und einem aus einem isolierendem Material bestehenden Hüllrohr besteht.

14. Zange nach Anspruch 13,
dadurch **gekennzeichnet,** daß das aus einem isolierendem Material bestehende Hüllrohr über eine Bajonettverbindung mit der eigentlichen Zange verbunden ist.

## Claims

1. Medical tongs, in particular, for use in endoscopic surgery, having
- a handpiece (1), provided with a stationary and a movable handle (11, 12),
- an external tube (2), which is connected to said stationary handle (11), and
- inside said external tube (2) a moveably guided draw-rod (3), which actuates at least one of the two tong elements (41, 42) of the tong jaws and which is connected to said movable handle (12),
**characterized** by a bayonet element (31), which can be inserted into a bayonet insert (24) inside said external tube (2) and to which at least one of said tong elements (41, 42) is joined, being borne on the distal end of said draw-rod (3) in such a manner that it can be moved relative to said draw-rod, and
by in said external tube (2) a spring clamp element (14, 15) which engages said draw-rod (3) in a formfitting manner when said handpiece is attached, being provided.

2. Tongs according to claim 1,
**characterized** by being able to connect said external tube (2) having said inserted draw-rod (3) to said handpiece (1) via a connection element (13,21), with the proximal end of said draw-rod (3) locking into a catch (51) in said movable handle (12) of said handpiece (1).

3. Tongs according to claim 2,
**characterized** by being able to move said catch (51) on said movable handle (12) by means of an actuating element (5) in such a manner that the opening of the tong jaws (4) can be set.

4. Tongs according to one of the claims 1 to 3,
**characterized** by said draw-rod (3) having at its proximal end section two flat sections (32) which face each other and into which said spring clamp element (14, 15) of said external tube (2) engages in a formfitting manner.

5. Tongs according to one of the claims 1 to 4,
**characterized** by said draw-rod (3) is designed spherical in shape at its proximal end with which it can be inserted into the holding part of said spring catch (51).

6. Tongs according to one of the claims 1 to 5,
**characterized** by a spring snapping said movable handle (12) into a position in which said tong elements (41, 42) are closed.

7. Tongs according to one of the claims 1 to 6,
**characterized** by the connection element which connects said external tube (2) to the handpiece (1) being a screw element (13, 21).

8. Tongs according to one of the claims 1 to 7,
**characterized** by said external tube (2) being rotatable relative to the part (21) of said connection element attached to said handpiece (1).

9. Tongs according to claim 8,
**characterized** by being able to set defined rotational positions between said external tube (2) and said handpiece (1).

10. Tongs according to claim 9,
**characterized** by a ball/catch connection defining said rotational positions.

11. Tongs according to one of the claims 1 to 10,
**characterized** by both said tong elements (41, 42) being movable.

12. Tongs according to claim 11,
**characterized** by said tong elements (41, 42) being connected to said draw-rod via pivotal elements (43, 44).

13. Tongs according to one of the claims 1 to 12,
**characterized** by said external tube (2) being composed of a metallic internal tube and a jacket tube made of an insulating material.

14. Tongs according to claim 13,
**characterized** by said jacket tube made of an insulating material being connected to the actual tongs via a bayonet joint.

## Revendications

1. Pince médicale notamment conçue pour l'utilisation dans des interventions endoscopiques, comprenant
- un embout de préhension (1) composé de poignées fixe et mobile (11, 12),
- un tube extérieur (2) relié à la poignée fixe (11), et
- une tige de traction (3) qui est guidée à coulissement dans le tube extérieur (2), actionne au moins l'un des deux éléments d'enserrement (41, 42) de la gueule de la pince, et est reliée à la poignée mobile (12),
caractérisée par le fait qu'un élément (31) à effet baïonnette, monté coulissant par rapport à la tige de traction (3) sur l'extrémité distale de ladite tige, peut être inséré dans une pièce intégrée (24) à effet baïonnette, logée dans le tube extérieur (2), et est articulé sur au moins l'un des éléments d'enserrement (41, 42) ; et
qu'un élément de coincement (14, 15), chargé par un ressort et prévu dans le tube extérieur (2), pénètre par concordance de formes dans la tige de traction (3) lorsque l'embout de préhension est mis en place.

2. Pince selon la revendication 1,
caractérisée par le fait que le tube extérieur (2), dans lequel la tige de traction (3) est intégrée, peut être relié à l'embout de préhension (1) par l'intermédiaire d'un élément de solidarisation (13, 21), l'extrémité proximale de la tige de traction (3) s'encliquetant dans une pièce (51) à déclic, dans la poignée mobile (12) de l'embout de préhension (1).

3. Pince selon la revendication 2,
caractérisée par le fait que la pièce (51) à déclic peut être animée de coulissements sur la poignée mobile (12), par l'intermédiaire d'un élément d'actionnement (5), de telle sorte que l'ouverture de la gueule (4) de la pince puisse être réglée.

4. Pince selon l'une des revendications 1 à 3,
caractérisée par le fait que la tige de traction (3) présente, dans sa région extrême proximale, deux méplats (32) tournés à l'opposé l'un de l'autre et dans lesquels pénètre, par concordance de formes, l'élément de coincement (14, 15) du tube extérieur (2) qui est chargé par ressort.

5. Pince selon l'une des revendications 1 à 4,
caractérisée par le fait que la tige de traction (3) est de réalisation sphérique à son extrémité proximale par laquelle elle peut être insérée dans le logement de la pièce (51) à déclic chargée par ressort.

6. Pince selon l'une des revendications 1 à 5,
caractérisée par le fait qu'un ressort sollicite la poignée mobile (12) vers la position dans laquelle les éléments d'enserrement (41, 42) sont fermés.

7. Pince selon l'une des revendications 1 à 6,
caractérisée par le fait que l'élément de solidarisation, reliant le tube extérieur (2) à l'embout de préhension (1), est un élément vissable (13, 21).

8. Pince selon l'une des revendications 1 à 7,
caractérisée par le fait que le tube extérieur (2) peut tourner par rapport à la partie (21) de l'élément de solidarisation qui est installée sur l'embout de préhension (1).

9. Pince selon la revendication 8,
caractérisée par le fait que des positions bien définies entre le tube extérieur (2) et l'embout de préhension (1), prises par rotation, peuvent être réglées.

10. Pince selon la revendication 9,
caractérisée par le fait qu'un système d'encliquetage à bille définit les positions prises par rotation.

11. Pince selon l'une des revendications 1 à 10,
caractérisée par le fait que les deux éléments d'enserrement (41, 42) sont mobiles.

12. Pince selon la revendication 11,
caractérisée par le fait que les éléments d'enserrement (41, 42) sont reliés à la tige de traction par l'intermédiaire d'éléments pivotants (43, 44).

13. Pince selon l'une des revendications 1 à 12,
caractérisée par le fait que le tube extérieur (2) se compose d'une tubulure intérieure métallique et d'une tubulure d'enveloppement en un matériau isolant.

14. Pince selon la revendication 13,
caractérisée par le fait que la tubulure d'enveloppement, constituée d'un matériau isolant, est reliée à la pince proprement dite par l'intermédiaire d'une solidarisation du type baïonnette.
